Europäisches Patentamt

European Patent Office

Office européen des brevets

(19)

(11) Veröffentlichungsnummer: **0 073 354**
**B1**

(12)

# EUROPÄISCHE PATENTSCHRIFT

(45) Veröffentlichungstag der Patentschrift:
05.12.84

(51) Int. Cl.³: **C 09 D 3/66, C 08 L 67/02**

(21) Anmeldenummer: **82107029.9**

(22) Anmeldetag: **04.08.82**

(54) Säuresterilisationsfeste Polyesterlacke.

(30) Priorität: **02.09.81 DE 3134640**

(43) Veröffentlichungstag der Anmeldung:
**09.03.83 Patentblatt 83/10**

(45) Bekanntmachung des Hinweises auf die Patenterteilung:
**05.12.84 Patentblatt 84/49**

(84) Benannte Vertragsstaaten:
**AT BE CH DE FR GB IT LI LU NL SE**

(56) Entgegenhaltungen:
**DE - A - 2 934 443**
**DE - A - 3 000 662**

(73) Patentinhaber: **DYNAMIT NOBEL AKTIENGESELLSCHAFT, Postfach 1209, D-5210 Troisdorf, Bez. Köln (DE)**

(72) Erfinder: **Schade, Gerhard, Dr., Akazienweg 13, D-5810 Witten (DE)**
Erfinder: **Schmitthenner, Martin, Dr., Kreisstrasse 68, D-5810 Witten (DE)**
Erfinder: **Vollkommer, Norbert, Dr., Schumannstrasse 5, D-5210 Troisdorf-Kriegsdorf (DE)**

Beschreibung

Die DE-AS Nr. 1807776 beschreibt kochfeste, sterilisierbare und hochverformbare Lackfilme aus hochmolekularen amorphen Mischpolyestern, die im wesentlichen aus Tere- und Isophthalsäure sowie Alkylenglykolen und 2,2-Dimethylpropandiol-1,3, 5 bis 30% eines Benzoguanaminharzes, 0,5 bis 2% saurer Katalysatoren und ggf. Pigmenten hergestellt wurden. Solche Lackfilme zeigen hohe Oberflächenhärte und zugleich gute Verformbarkeit. Tiefgezogene Verpackungsartikel aus Feinblech, vorbeschichtet mit solchen Lackfilmen, sind ohne Lackierungsschäden wie Haftungsverlust an verformten Stellen oder Wasseraufnahme in Wasser bei 121°C sterilisierbar.

Die DE-AS Nr. 2126048 beschreibt Lackfilme, die gegenüber der DE-AS Nr. 1807776 im wesentlichen gleiche chemische Zusammensetzung, aber niedrige Glasumwandlungspunkte zwischen 30 und 50°C und relative Viskositäten von 1,5 bis 1,8 aufweisen und noch gesteigerte Verformbarkeit ergeben.

Die DE-AS Nr. 2521792 beschreibt Überzugsmittel, die Aminoplaste und Polyester aus einerseits Tere- bzw. Isophthalsäure und 5 bis 50 Mol-% Hexahydroterephthalsäure sowie andererseits aliphatische Diole und 2,2-Dimethylpropandiol-1,3 enthalten. Filme aus diesen Überzugsmitteln weisen zwar hinlänglich gute Verformbarkeit auf, zeigen jedoch unter Sterilisationsbedingungen im Kontakt mit Säuren zu geringe Beständigkeit.

Die DE-AS Nr. 2521791 beschreibt Überzugsmittel, die Aminoplaste und Polyester aus einerseits Tere- bzw. Isophthalsäure und andererseits aliphatische Diole, 2,2-Dimethylpropandiol-1,3 und 1,4-Bishydroxymethylcyclohexan enthalten. Diese Überzugsmittel liefern Lackfilme mit hinlänglich guter Verformbarkeit, aber zu geringer Beständigkeit unter Sterilisationsbedingungen im Kontakt mit Säuren.

Die DE-OS Nr. 2934443 beschreibt Überzugsmittel, die Aminoplaste und Polyester aus einerseits aromatischen sowie ggf. aliphatischen und cycloaliphatischen Dicarbonsäuren und andererseits aliphatische Diole, cycloaliphatische Diole und 2,2-Dimethylpropandiol-1,3 sowie 5 bis 50 Mol-% des Diolanteils Bis(hydroxymethyl)-tricyclo-(5.2.1.0$^{2,6}$)-decan enthalten. Diese Überzugsmittel weisen ausreichende Verformbarkeit, aber geringe Beständigkeit unter Sterilisationsbedingungen im Kontakt mit Säuren auf.

Überzugsmittel nach diesem Stand der Technik zeigen unter Sterilisationsbedingungen im Kontakt mit Säuren keine oder geringe Beständigkeit, wodurch der Einsatz als Lackierung eingeschränkt und die Verwendung als Innenschutzlack für Verpackungen sterilisierter saurer Nahrungsmittel nicht möglich ist. Zwar war es möglich, durch Variation der Diole und Dicarbonsäuren Polyester zu Lackharzen zu verarbeiten, die sich als Lackierung von Blechen als genügend verformbar bei der Herstellung von z.B. Dosenverpackungen und als kochfest bei der Sterilisierung von neutralem Füllgut erwiesen, jedoch gab es keinen Hinweis, dass Lackierungen auf der Basis von Polyester gegen den Säuregehalt saurer Nahrungsmittel ohne Schäden bzw. Ablösung der Lackierung vom Blech beständig sein konnten. Es war daher nicht möglich, Verpackungen für saure Nahrungsmittel wie Essigkonserven, Fruchtsäfte und -konzentrate, Sauerkraut, Fischkonserven mit Gehalten von Essig usw. mit Überzügen auf Basis von Polyestern zu versehen.

Es bestand die Aufgabe, hochverformbare Lackfilme auf Basis hochmolekularer, linearer, gesättigter Polyester herzustellen, die unter Sterilisationsbedingungen im Kontakt mit Säuren genügende Beständigkeit und Haftung am Basismaterial der Verpackung aufweisen.

Überraschend wurde gefunden, dass diese Aufgabe mit Überzugsmitteln auf der Basis von gesättigten, linearen Polyestern gelöst werden kann, deren Diolanteil zu 100 bis 70 Mol-% aus Bis(hydroxymethyl)tricyclodecan und deren Dicarbonsäureanteil aus Terephthalsäure und/oder Isophthalsäure und ggf. bis zu 10 Mol-% weiterer Dicarbonsäuren besteht. Die Beständigkeit gegen Säuregehalte der Füllgüter ist unter Sterilisationsbedingungen ausgezeichnet, im Gegensatz zu der geringen Beständigkeit von Überzügen auf Basis von hierfür bekannten, chemisch nahestehender Polyester.

Gegenstand der Erfindung sind daher hitzehärtende Überzugsmittel, bestehend aus Bindemitteln, Lösemitteln sowie ggf. üblichen Lackzusätzen wie Pigmenten, Füllstoffen und Lackhilfsstoffen, die als Bindemittel:

a) 5 bis 35 Gew.-Teile Triazinharze, vorzugsweise Benzoguanaminharze, oder verkappte Polyisocyanatharze, und

b) 95 bis 65 Gew.-Teile gesättigter, linearer Polyester mit relativen Viskositäten zwischen 1,3 und 1,8 mit einem Dicarbonsäureanteil aus Resten der Terephthal- und/oder Isophthalsäure sowie ggf. bis zu 10 Mol-% aus Resten weiterer Dicarbonsäuren und einen Diolanteil aus Bis(hydroxymethyl)tricyclodecan und weiteren Diolen,

welche dadurch gekennzeichnet sind, dass der Diolanteil aus 100 bis 70 Mol-% Bis(hydroxymethyl)tricyclodecan und 0 bis 30 Mol-% weiterer Diolen besteht.

Bis(hydroxymethyl)tricyclodecan ist an sich als Bestandteil linearer Polyester aus der DE-OS Nr. 1495667 bekannt; darin verwies aber nichts auf die überlegenen Eigenschaften von Überzugsmitteln, wenn der Gehalt im Polyester 70 Mol-% des Diolanteils erreicht oder überschreitet. Bis(hydroxymethyl)tricyclodecan, im folgenden kurz TCD-Diol genannt, ist ein Gemisch zahlreicher Isomere, in denen zwei Hydroxymethylgruppen an verschiedenen Positionen der Ringe stehen können und die Ringe verschiedene Raumformen zueinander haben können.

Der Anteil von 0 bis 30 Mol-% der Diolkomponente kann aus beliebigen aliphatischen oder cycloaliphatischen Diolen bestehen. Bevorzugt besteht der Anteil ganz oder überwiegend aus 2,2-Dimethylpropandiol-1,3. Kleinere Anteile von bis etwa 4 Gew.-% von einkondensiertem Schleppdiol kommen vor und stören nicht. Als Dicarbonsäure ist Terephthalsäure bevorzugt. Weiter bevorzugt sind Anteile bis 50 Mol-% Isophthalsäure neben Terephthalsäure. Anteile weiterer aromatischer, aliphatischer oder cycloaliphatischer Dicarbonsäuren sind möglich, aber nicht bevorzugt.

Die erfindungsgemäss verwendeten Polyester werden nach bekannten Verfahren, vorzugsweise unter Einsatz von Kondensationskatalysatoren hergestellt. Derartige Verfahren sind beispielsweise in W.R. Sorenson, T.W. Campbell, ,,Preparative Methods of Polymer Chemistry'', Interscience Publishers, Inc., New York 1961, s. 111 bis 127, 298 bis 304, beschrieben.

Die erfindungsgemäss verwendeten Polyester weisen eine relative Viskosität von 1,3 bis 1,8 auf, wobei als relative Viskosität das Verhältnis $\eta_{rel} = t_1/t_0$ ist, worin $t_1$ die Durchlaufzeit einer Lösung von 1 g Polymer in 100 ml eines Gemisches aus 60 Gew.-Teilen Phenol und 40 Gew.-Teilen 1,1,2,2-Tetrachloräthan und $t_0$ die Durchlaufzeit des Lösemittelgemisches in einem Kapillarviskometer bei 25°C bedeuten.

Die Polyester weisen Glastemperaturen von mindestens 90 bis 95 bis hinauf zu etwa 130°C auf. Die Polyester der Beispiele 1, 2 und 3 zeigten Glasumwandlung in den Temperaturbereichen 123 bis 130°C, 114 bis 121°C und 111 bis 117°C. Der Polyester gemäss Beispiel 4 zeigte eine noch befriedigende Glasumwandlungstemperatur im Bereich um 98°C. Die hohen Glasumwandlungstemperaturen der Polyester lassen an sich geringe Verformbarkeit der hieraus formulierten Überzugsmittel erwarten. Die gefundene gute Verformbarkeit der Überzugsmittel (vgl. die Schlagverformbarkeit nach der Tabelle) ist daher überraschend.

Die Hydroxylzahl der Polyester ist gering mit Werten unter 5 im oberen Bereich der relativen Viskositäten und Werten von maximal etwa 20 im unteren Viskositätsbereich.

Die Polyester sind überraschend gut löslich in verschiedenen, üblichen Lacklösemitteln, beispielsweise in Gemischen aromatischer Kohlenwasserstoffe des Siedebereichs 150 bzw. 200°C. Überraschend ist besonders die sehr gute Löslichkeit von Polyestern mit Terephthalsäure als einziger Säurekomponente wie auch die hohe Löslichkeit bei alleinigem oder sehr hohem Anteil TCD-Diol, die durch anteilige Mitverwendung von 2,2-Dimethylpropandiol-1,3 nicht weiter verbesserbar ist. Damit steht die gute Löslichkeit im Gegensatz zu der geringen Löslichkeit entsprechender Polyester auf Basis üblicher Diole, besonders auch auf Basis von 1,4-Hydroxymethylcyclohexan.

Als Triazinharze werden vorzugsweise Benzoguanaminharze eingesetzt, jedoch sind auch Melaminharze, ggf. in Abmischungen mit Triazinoder Benzoguanaminharzen verwendbar. Vorzugsweise werden 25 bis 35 Gew.-Teile Benzoguanaminharze, im Falle der Triazinharze vorzugsweise 15 bis 25 Gew.-Teile verwendet. Verkappte Polyisocyatharze werden vorzugsweise mit Polyestern nach b im molaren Verhältnis der -NCO-Gruppen der Polyisocyanate zu den OH-Gruppen der Polyester von 0,5 bis 1,25 zu 1 verwendet. Verkappte Polyisocyanate sind solche, die durch Reaktion mit geeigneten Blockierungsmitteln, beispielsweise Oximen oder Caprolactam an vorzeitiger Reaktion mit Hydroxygruppen der Polyester bei Normaltemperatur gehindert sind und erst bei Temperaturen der Hitzehärtung aufspalten und reaktionsfähig werden.

Für die Herstellung der erfindungsgemässen Überzugsmittel werden bevorzugt den Ausgangslacklösungen wirkungsvolle Härtungskatalysatoren zugesetzt. Für Triazinharze kommen besonders Schwefelsäure, Phosphorsäure, Salzsäure, Oxalsäure, Benzolsulfonsäure und homologe, alkylierte Benzolsulfonsäuren wie z.B. Dodecylbenzolsulfonsäure od. dgl. sowie deren Salze mit bei höheren Temperaturen flüchtigen Basen, wie Ammoniak, Mono-, Di- und Trialkylamine, Morpholin, 2-Amino-2-methylpropanol-1 usw. in Frage. Für verkappte Polyisocyanatharze sind als Härtungskatalysatoren besonders organische Zinnverbindungen, insbesondere Dibutylzinndilaurat oder andere für die Polyurethanbildung übliche Katalysatoren verwendbar.

Als Füllstoffe und/oder Pigmente kommen u.a. Titandioxid, Kreide, Kieselsäure, Magnesiumoxid und Pigmentfarbstoffe in üblicher Menge in Frage. Weiterhin können Lackhilfsstoffe wie beispielsweise Netzmittel, Entschäumer, Thixotropierungsmittel usw. eingesetzt werden.

Die erfindungsgemässen Überzugsmittel werden nach bekannten Verfahren aufgetragen, vorzugsweise durch Walzen oder Spritzen.

Die Erfindung beschränkt sich nicht nur auf Beschichtung von metallischen Substraten mit den erfindungsgemässen, hitzehärtenden Überzugsmitteln, sondern erstreckt sich auch auf weitere temperaturbeständige Untergründe, wie beispielsweise keramische oder gläserne Stoffe. Selbst die Beschichtung temperaturbeständiger Kunststoffe, insbesondere Duroplaste, mit den erfindungsgemässen Überzugsmitteln ist möglich.

Die gemäss vorangehenden Ausführungen hergestellten Überzugsmittel zeigen vorzugsweise für den Einsatz als Innenschutzlacke von Verpackungsmitteln, sogenannten Goldlacken und Weisslacken, ausgezeichnete Eigenschaften, insbesondere hinsichtlich Sterilisationsbeständigkeit im Kontakt mit Säuren, daneben gute Schlagverformbarkeit, Oberflächenhärte, Abdruckfestigkeit, Haftvermögen, insbesondere auf metallischen Substraten.

In den nachfolgenden Beispielen werden zur Charakterisierung von erfindungsgemässen und bekannten Überzugsmitteln folgende Prüfmethoden verwendet.

1. *Sterilisationsbeständigkeit*

Aus lackiertem Blechabschnitt (z.B. Weissblech, 0,25 mm, E 1, Passivierung 311 oder Aluminium, 0,25 mm, anod. passiviert) wird eine Konservendose ausgestanzt. Die tiefgezogene Dose wird auf Sterilisationsbeständigkeit (Prüfverfahren des Instituts für Lebensmitteltechnologie und Verpackung der TU München, Merkblatt 11, ,,

Prüfverfahren für Konservendosenlacke", Teil 7: „Verpackungs-Rundschau", *30* (1979) Nr. 5, S. 38), jedoch bei 121°C 1 bar Überdruck, 60 min mit den in der Tabelle genannten Prüfmedien geprüft. Darin sind c und d Härtetests mit Säure erhöhter Konzentration, die eine stärkere Differenzierung der Lackbeständigkeit erlauben. In der Bewertungsskala bedeuten: 0 einwandfrei, keine Ätzung oder Mattierung; 1 geringe Mattierung; 2 brauchbar, geringe Ätzung; 3 unbrauchbar, Ätzung bzw. Stellen mit Haftverlust des Lackes an der Dose; 4 bis 5 zunehmende Ätzung bzw. zunehmender Haftverlust; 6 bis 10 Dosenblech im steigenden Masse korrodiert, starke bis sehr starke Ätzung bzw. Haftverlust.

    2. *Verformbarkeit*
    a) *Schlagverformbarkeit*
*Wedge-bend*-Test (Prüfverfahren des Instituts für Lebensmitteltechnologie und Verpackung an der TU München, Merkblatt 11, „Prüfverfahren für Konservendosenlacke", Teil 5: „Verpackungs-Rundschau", *25* (1974) Nr. 6 Techn.-wiss. Beilage, S. 47/48); 0 mm bester Wert, 100 mm schlechtester Wert.

    b) *Erichsen-Näpfchen (langsame Verformung)*
Einziehen einer Sicke (mm) in den Mantel des tiefgezogenen Näpfchens bis zur beginnenden Rissbildung auf der Sicken-Kuppe.

Der Vergleichbarkeit wegen haben die Beispiele 1 bis 5 und die Vergleichsbeispiele A bis C vergleichbare Rezepturen und gleiche Verarbeitung; die nachfolgenden Beispiele erläutern weiter die Breite der Erfindung.

*Beispiel 1:*

18,8 Gew.-Teile eines Polyesters, der aus Terephthalsäure und TCD-Diol und einer überwiegend später wieder entfernten Menge Äthylenglykol als Schleppdiol hergestellt wurde und eine relative Viskosität von 1,70 aufweist, werden in 43,9 Gew.-Teilen einer Mischung aus Solvesso 150 $^{\text{R}}$ und Solvesso 200 $^{\text{R}}$ im Gew.-Verhältnis 4:1 gelöst und mit 23,3 Gew.-Teilen TiO$_2$ (Rutil-Type) pigmentiert. Diese Pigmentpaste wird mit einer Perlmühle vermahlen. Anschliessend wird mit 12,3 Gew.-Teilen eines handelsüblichen Benzoguanaminharzes (Maprenal MF 980/62%ige Lieferform in n-Butanol) aufgelackt und mit 0,3 Gew.-Teilen Dodecylbenzolsulfonsäure als Säurekatalysator (Catalyst 600) versetzt. Der fertige Lack wird mit einer Spiralrakel auf einen Blechabschnitt aus Weissblech, 0,25 mm, E 1, Passivierung 311, aufgezogen und bei 210°C 15 min eingebrannt (Trockenschichtdicke ca. 10 µm).

*Beispiel 2:*

Der Lackansatz und die Lackierung des Beispiels 1 wird wiederholt, wobei der Polyester gleiche Zusammensetzung, jedoch eine relative Viskosität von 1,53 aufweist.

*Beispiel 3:*

Der Lackansatz und die Lackierung des Beispiels 1 wird wiederholt, wobei der Polyester gleiche Zusammensetzung, jedoch eine relative Viskosität von 1,34 aufweist.

*Beispiel 4:*

Der Lackansatz und die Lackierung des Beispiels 1 wird wiederholt, wobei der Polyester einerseits jedoch aus Terephthalsäure, andererseits aus einem Dialkoholgemisch aus TCD-Diol und 2,2-Dimethylpropandiol (1,3) im Mol-Verhältnis 4:1 besteht und eine relative Viskosität von 1,63 aufweist.

*Beispiel 5:*

Der Lackansatz und die Lackierung des Beispiels 1 wird wiederholt, wobei der Polyester einerseits aus einem Dicarbonsäuregemisch aus Terephthalsäure und Isophthalsäure im Mol-Verhältnis 1:1 und andererseits aus TCD-Diol besteht und eine relative Viskosität von 1,50 aufweist.

*Vergleichsbeispiel A:*

Beispiel 1 wird mit der im Beispiel 1 angegebenen Lackrezeptur, jedoch mit einem Polyester aus einem Dicarbonsäuregemisch, bestehend aus Terephthalsäure und Isophthalsäure im Mol-Verhältnis 1:1 und einem Dialkoholgemisch, bestehend aus 2,2-Dimethylpropandiol-1,3 und Äthylenglykol im Mol-Verhältnis 1:1 und einer relative Viskosität von 1,65 (entsprechend DE-AS Nr. 1807776) wiederholt.

*Vergleichsbeispiel B:*

Beispiel 1 wird mit der im Beispiel 1 angegebenen Lackrezeptur, jedoch mit einem Polyester aus einem Dicarbonsäuregemisch, bestehend aus Terephthalsäure und Isophthalsäure im Mol-Verhältnis 9:11 und einem Dialkoholgemisch, bestehend aus 1,4 Hydroxymethylcyclohexan, 2,2-Dimethylpropandiol-1,3 und Äthylenglykol im Mol-Verhältnis 25:70:5 und einer relativen Viskosität von 1,68 (entsprechend DE-AS Nr. 2521791) wiederholt.

*Vergleichsbeispiel C:*

Beispiel 1 wird mit der im Beispiel 1 angegebenen Lackrezeptur, jedoch mit einem Polyester aus einem Dicarbonsäuregemisch, bestehend aus Terephthalsäure und Isophthalsäure im Mol-Verhältnis 3:7 und einem Dialkoholgemisch, bestehend aus TCD-Diol sowie 2,2-Dimethylpropandiol-1,3 im Mol-Verhältnis 1:3 sowie einer geringen Menge Äthylenglykol als Schleppdiol und einer relative Viskosität von 1,64 (entsprechend DE-OS Nr. 2934443) wiederholt.

*Beispiel 6:*

20,0 Gew.-% eines Polyesters aus Terephthalsäure und TCD-Diol, hergestellt entsprechend Beispiel 1, jedoch mit einer relativen Viskosität von 1,41, werden in 50,7 Gew.-% einer Mischung von Solvesso 150 $^{\text{R}}$ und Solvesso 200 $^{\text{R}}$ im Gew.-Verhältnis 4:1 gelöst und mit 25,8 Gew.-Teilen TiO$_2$ pigmentiert und zu einer Paste vermahlen. Darauf wird mit 3,4 Gew.-% Teilen eines handels-

süblichen blockierten Polyisocyanatharzes (IPDI-Addukt B 1370/60%ige Lieferform in Butylacetat/Phenol; Hersteller Veba-Hüls) und mit 0,1 Gew.-% Dibutylzinndilaurat versetzt. Es wird 12 min bei 160°C zu einer Schicht von ca. 10 µm eingebrannt.

*Tabelle*

| Beispiel | 1 | 2 | 3 | 4 | 5 | 6 | A | B | C |
|---|---|---|---|---|---|---|---|---|---|
| *Sterilisationsbeständigkeit* | | | | | | | | | |
| a. Wasser, neutral | 0 | 0 | 0 | 0 | 0 | 0 | 0 | 0 | 0 |
| b. Milchsäure,  1 gew.-%ig | 0-1 | 0-1 | 0-1 | 1 | 0-1 | 0-1 | 3 | 3 | 3 |
| c. dto.           2 gew.-%ig | 1 | 1 | 1 | 1-2 | 1 | 1-2 | 6 | 6 | 6 |
| d. dto.           3 gew.-%ig | 2 | 2 | 2 | 2-3 | 2 | 2-3 | 10 | 10 | 10 |
| e. Citronen-/Weinsäure     1 gew.-%ig | 0-1 | 0-1 | 0-1 | 1 | 0-1 | 0-1 | 3 | 3 | 3 |
| f. Kochsalz/Essigsäure     2 gew.-%ig | 0 | 0 | 0 | 0 | 0 | 0 | 1 | 1 | 1 |
| *Verformbarkeit* | | | | | | | | | |
| Schlagverformbarkeit (mm) | 40 | 35 | 40 | 50 | 60 | 35 | 68 | 53 | 45 |
| Erichsen-Näpfchen (mm) | 1,0 | 1,0 | 0,7 | 0,5 | 0,5 | 0,5 | 0,5 | 0,5 | 0,5 |
| Prüfmedien b bis f: Verdünnungsmittel Wasser | | | | | | | | | |

**Patentansprüche** für die Vertragsstaaten BE, CH, DE, FR, GB, IT, LI, LU, NL, SE

1. Hitzehärtende Überzugsmittel, bestehend aus Bindemitteln, Lösemittel sowie gegebenenfalls üblichen Lackzusätzen wie Pigmenten, Füllstoffen und Lackhilfsstoffen, die als Bindemittel:
a) 5 bis 35 Gew.-Teile Triazinharze, vorzugsweise Benzoguanaminharze, oder verkappte Polyisocyanatharze, und
b) 95 bis 65 Gew.-Teile gesättigter, linearer Polyester mit relativen Viskositäten zwischen 1,3 und 1,8 mit einem Dicarbonsäureanteil aus Resten der Terephthal- und/oder Isophthalsäure sowie ggf. bis zu 10 Mol-% aus Resten weiterer Dicarbonsäuren und einen Diolanteil aus Bis(hydroxymethyl)tricyclodecan und weiteren Diolen enthalten,
dadurch gekennzeichnet, dass der Diolanteil aus 100 bis 70 Mol-% Bis(hydroxymethyl)tricyclodecan und 0 bis 30 Mol-% weiteren Diolen besteht.
2. Überzugsmittel nach Anspruch 1, dadurch gekennzeichnet, dass der weitere Diolanteil ganz oder überwiegend aus 2,2-Dimethylpropandiol-1,3 besteht.
3. Verwendung der Überzugsmittel nach den vorangehenden Ansprüchen als Lack auf bevorzugt metallischen Untergründen insbesondere als Innenlack von Verpackungsmitteln.

**Patentansprüche** für den Vertragsstaat: AT

1. Verfahren zur Herstellung hitzehärtender Überzugsmittel in an sich bekannter Weise aus Bindemitteln, Lösemittel sowie gegebenenfalls üblichen Lackzusätzen, wie Pigmenten, Füllstoffen und Lackhilfsstoffen, wobei als Bindemittel:

a) 5 bis 35 Gew.-Teile Triazinharze, vorzugsweise Benzoguanaminharze, oder verkappte Polyisocyanatharze, und
b) 95 bis 65 Gew.-Teile gesättigter, linearer Polyester mit relativen Viskositäten zwischen 1,3 und 1,8 mit einem Dicarbonsäureanteil aus Resten der Terephthal- und/oder Isophthalsäure sowie gegebenenfalls bis zu 10 Mol-% aus Resten weiterer Dicarbonsäuren und einem Diolanteil aus Bis(hydroxymethyl)tricyclodecan und weiteren Diolen enthalten sind,
dadurch gekennzeichnet, dass der Diolanteil aus 100 bis 70 Mol-% Bis(hydroxymethyl)tricyclodecan und 0 bis 30 Mol-% weiteren Diolen besteht.
2. Verfahren nach Anspruch 1, dadurch gekennzeichnet, dass der weitere Diolanteil ganz oder überwiegend aus 2,2-Dimethylpropandiol-1,3 besteht.
3. Verwendung der nach den vorangehenden Ansprüchen hergestellten Überzugsmittel als Lack auf bevorzugt metallischen Untergründen, insbesondere als Innenlack von Verpackungsmitteln.

**Revendications** pour les Etats contractants BE, CH, DE, FR, GB, IT, LI, LU, NL, SE

1. Revêtement durcissant à chaud constitué par des liants, des solvants ainsi qu'éventuellement par les additifs usuels pour vernis tels que pigments, charges et adjuvants pour peintures, renfermant, en tant que liant:
a) 5 à 35 parties en poids de résines de triazine, de préférence de résines de benzoguanamine ou de résines de polyisocyanate masquées, et
b) 95 à 65 parties en poids de polyesters linéaires saturés avec des viscosités relatives entre 1,3 et 1,8, une fraction acide dicarboxylique composée de restes d'acide téréphtalique et/ou isophtalique, ainsi qu'éventuellement jusqu'à 10% molaires de restes d'autres acides dicarboxyliques, et une fraction diol composée de bis-(hydroxyméthyl)tricyclodécane et d'autres diols, caractérisé en ce que la fraction diol est constituée de 100 à 70% molaires de bis(hydroxyméthyl)-tricyclodécane et de 0 à 30% molaires d'autres diols.

2. Revêtement selon la revendication 1, caractérisé en ce que l'autre fraction diol est constituée en totalité ou en prédominance par du 2,2-diméthylpropanediol-1,3.

3. Utilisation des revêtements selon les revendications précédentes comme vernis, de préférence sur des supports métalliques, en particulier comme vernis intérieur pour matières d'emballage.


**Revendications** pour l'Etat contractant: AT

1. Procédé de préparation de produits de revêtement durcissant à la chaleur, d'une manière connue en soi, à partir de liants, de solvants, et éventuellement des additifs courants pour vernis, comme des pigments, charges et adjuvants de vernis, où on utilise en tant que liants:

a) 5 à 35 parties en poids de résines de triazine, de préférence de résines de benzoguanamine, ou des résines polyisocyanates bloquées en bout, et

b) 95 à 65 parties en poids de polyesters linéaires saturés ayant une viscosité relative comprise entre 1,3 et 1,8, avec une partie acide dicarboxylique constituée de résidus de l'acide téréphtalique et/ou de l'acide isophtalique, et éventuellement jusqu'à 10% molaires de résidus d'autres acides dicarboxyliques, et une partie diol constituée de bis(hydroxyméthyl)tricyclodécane et d'autres diols,
caractérisé en ce que la partie diol est constituée de 100 à 70% molaires de bis(hydroxyméthyl)-tricyclodécane et de 0 à 30% molaires d'autres diols.

2. Procédé selon la revendication 1, caractérisé en ce que la partie autres diols est constituée en totalité ou en majorité de 2,2-diméthyl-propanediol-1,3.

3. Utilisation des produits de revêtement préparés selon les revendications ci-dessus, en tant que vernis sur des subjectiles de préférence métalliques, en particulier en tant que vernis intérieur de produits d'emballage.


**Claims** for the contracting States BE, CH, DE, FR, GB, IT, LI, LU, NL, SE

1. Heat-hardening covering material consisting of binders, solvents as well as optionally the usual lacquer additives such as pigments, fillers and lacquer auxiliaries, which contains as binders:

(a) 5 to 35 parts by weight of triazine resins, preferably benzoguanamine resins, or masked polyisocyanate resins, and

(b) 95 to 65 parts by weight of saturated linear polyesters with relative viscosities between 1.3 and 1.8, with a content of dicarboxylic acids formed from residues of terephthalic and/or isophthalic acid, as well as optionally up to 10 mol-% of the residues of further dicarboxylic acids and a diol content formed from bis-(hydroxymethyl)-tricyclodecane and further diols, characterised in that the diol content consists of 100 to 70 mol-% of bis-(hydroxymethyl)-tricyclodecane and 0 to 30 mol-% of further diols.

2. Covering material according to Claim 1, characterised in that the further diol content consists completely or predominantly of 2,2-dimethylpropanediol-1,3.

3. Use of the covering material according to the preceding claims, as lacquer on preferably metallic substrates, especially as inner lacquer of packagings.


**Claims** for the contracting State: AT

1. Process for the production of heat-hardening covering material in conventional manner from binders, solvents as well as optionally the usual lacquer additives, such as pigments, fillers and lacquer auxiliaries, with there being contained as binder:

(a) 5 to 35 parts by weight of triazine resins, preferably benzoguanamine resins, or masked polyisocyanate resins, and

(b) 95 to 65 parts by weight of saturated linear polyesters with relative viscosities between 1.3 and 1.8, with a content of dicarboxylic acids formed from residues of terephthalic and/or isophthalic acid, as well as optionally up to 10 mol-% of the residues of further dicarboxylic acids and a diol content formed from bis-(hydroxymethyl)-tricyclodecane and further diols, characterised in that the diol content consists of 100 to 70 mol-% of bis-(hydroxymethyl)-tricyclodecane and 0 to 30 mol-% of further diols.

2. Process according to Claim 1, characterised in that the further diol content consists completely or predominantly of 2,2-dimethylpropanediol-1,3.

3. Use of the covering material according to the preceding claims, as lacquer on preferably metallic substrates, in particular as inner lacquer of packagings.